(19) Europäisches Patentamt
European Patent Office
Office européen des brevets

(11) Publication number: 0 015 629
B1

(12) # EUROPEAN PATENT SPECIFICATION

(45) Date of publication of patent specification: 02.01.86

(51) Int. Cl.$^4$: C 07 F 7/10 // C07D501/24, C07D501/00

(21) Application number: 80200215.4

(22) Date of filing: 06.03.80

(54) **New intermediates for the preparation of cephalosporins and process for the preparation of the intermediates.**

(30) Priority: 06.03.79 NL 7901774

(43) Date of publication of application:
17.09.80 Bulletin 80/19

(45) Publication of the grant of the patent:
02.01.86 Bulletin 86/01

(84) Designated Contracting States:
AT BE CH DE FR GB IT LU NL SE

(56) References cited:
EP-A-0 001 149
FR-A-2 059 686

(73) Proprietor: Gist - Brocades N.V.
Wateringseweg 1 P.O. Box 1
NL-2600 MA Delft (NL)

(72) Inventor: Bruynes, Cornelis Adrianus
Ridderhoflaan 51
NL-2396 CK Koudekerk a/d Rijn (NL)

(74) Representative: Huygens, Arthur Victor, Dr. et al
c/o Gist-Brocades N.V. Patent & Trademarks
Department Wateringseweg 1 PO-box 1
NL-2600 MA Delft (NL)

**The file contains technical information submitted after the application was filed and not included in this specification**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

Courier Press, Leamington Spa, England.

**Description**

This invention relates to new bromo-substituted deacetoxycephalosporins which are suitable intermediates in processes for the preparation of known and new antibiotics.

These new compounds may be represented by the general formula:

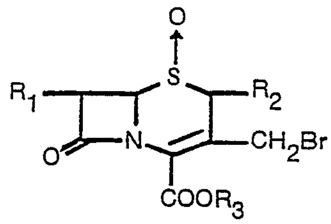

I

wherein $R_1$ represents a benzamido, formamido, phenylacetamido or phenoxyacetamido group, $R_2$ represents a hydrogen or bromine atom and $R_3$ represents a silyl group of the general formula:

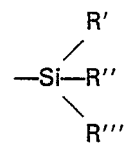

II

wherein R′, R″ and R‴ are the same or different and each represents an alkyl group (having at most 6 carbon atoms), a halogen-substituted alkyl group (having at most 6 carbon atoms) or an aryl group. The substituents in the positions 1 and 7 are in the β-configuration.

Compounds of the general type of formula I have been described before in the literature, a.o. in Dutch patent applications NL 70 04479, NL 70 12532 and NL 72 12992, wherein $R_2$ usually represents a hydrogen atom and $R_3$ is defined as the radical of an ester which can easily be hydrolyzed. In the first-mentioned application this radical is always derived from ordinary carbon esters, in the two other applications the possibility of silyl protection for the relevant carboxyl group has been indicated indeed, but has hardly ever been actually used. Compounds of formula I wherein $R_3$ represents a silyl group of formula II, have not been mentioned or characterized explicitly in these applications at all.

Still, the use of silyl protection in compounds of the type mentioned herebefore has clear advantages over the use of the conventional carbon protection, mainly because the protecting silyl group can be removed quite simply again, viz. by hydrolysis *in situ*, for example directly after the replacement of the bromine atom in the 3′-position by an other substituent has been effected, as is described in more detail herebelow.

The 3′-bromo-substituted compounds of formula I wherein $R_2$ is a hydrogen atom, are suitable intermediates in processes for the preparation of known and new antibiotics because the bromine atom in the 3′-position can be replaced in known and usually simple manner by a nucleophilic substituent, which substituents cannot be introduced directly into the 3-methyl group of corresponding unsubstituted deacetoxycephalosporins.

The 2,3′-dibromo-substituted deacetoxycephalosporins of formula I, wherein consequently $R_2$ is a bromine atom, can be partially debrominated in comparatively simple manner, thus replacing the bromine atom in the 2-position by a hydrogen atom, as has been described in European patent application 78200174 (EP—A—0001149). Thus, the 2,3′-dibromo-substituted deacetoxycephalosporins of formula I can also be used — after a preceding selective debromination — as intermediates in processes for the preparation of 3′-nucleophilic-substituted deacetoxycephalosporins.

The replacement of the bromine atom in the 3′-position in compounds of formula I (as the case may be, after previous removal of a bromine atom in the 2-position) by various types of nucleophilic groups, providing antibiotically valuable or intermediates for antibiotically valuable cephalosporins, has already been described extensively in the literature, a.o. in Dutch patent application NL 70 12532. Moreover, the compounds of formula I can also be used in processes for the preparation in conventional manner of new 3′-nucleophilic-substituted cephalosporins.

The new compounds of formula I may also be prepared in known manner. Suitable starting materials are for example, the corresponding unsubstituted unprotected cephalosporin sulphoxides of the general formula:

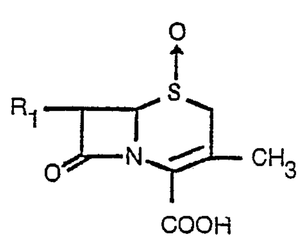

III

wherein $R_1$ is as hereinbefore defined.

These unprotected compounds are preferably first converted in conventional manner in a corresponding compound wherein the carboxy group is protected by a group which can easily be split off, such as the t-butyl group, before the compounds are subjected to a bromination reaction.

The latter reaction can be carried out for example, with one of the conventional bromination agents, such as N-bromosuccinimide, in a suitable organic medium and irradiation with ultraviolet or visible light.

Such bromination reactions usually provide mixtures of the corresponding 3'-bromo and 2,3'-dibromo-substituted compounds of the general formula:

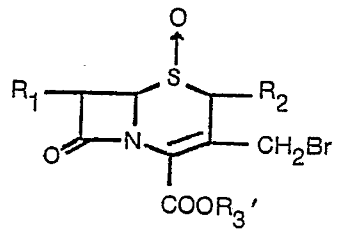

IV

wherein $R_1$ and $R_2$ are as hereinbefore defined and $R_3'$ represents a carboxyl-protecting group.

The ratio between the 3'-bromo and the 2,3'-dibromo-substituted products in the reaction mixture is determined by the starting materials and by the other conditions applied during the bromination reaction.

Both the bromo-substituted products can be obtained separately from the reaction mixture by applying for example, preparative high pressure chromatography on silica gel. If desired, the 2,3'-dibromo-substituted products can be converted (before, as well as after the separation from the bromination reaction mixture) into the corresponding 2-unsubstituted 3'-bromo-substituted compounds of formula IV wherein $R_2$ represents a hydrogen atom, by means of the method described in the European application 78200174.7 (EP—A—0001149).

The bromo-substituted products of formula IV wherein $R_1$ and $R_{3'}$ are as hereinbefore defined and $R_2$ represents a hydrogen or bromine atom, thus obtained can subsequently be hydrolyzed in known manner, for example by means of trifluoroacetic acid, and the corresponding compounds with a free carboxyl group of the formula:

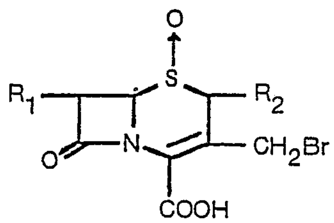

V

wherein $R_1$ and $R_2$ are as hereinbefore defined, thus obtained can then be silylated in known manner, thus providing the new compounds of formula I (the compounds of formula V wherein $R_2$ represents a bromine atom, are new compounds as well).

The preparation of the compounds of formula I is further illustrated by the following examples. Unless indicated otherwise, the IR-spectra were recorded as dispersions in KBr. The NMR-spectra have been measured at 60 MHz (unless stated otherwise), using tetramethyl silane as an internal standard; chemical shifts are indicated in δ values.

Example I

a. A mixture consisting of 103.2 g of dicyclohexylcarbodiimide, 43.5 g of t-butanol and 1.1 g of cuprous chloride was stored for 3 days at room temperature. The obtained compound was dissolved in 200 ml of dichloromethane and the solution was added dropwise to a stirred suspension of 35 g of 7-benzamido-3-methyl-3-cephem-4-carboxylic acid 1-oxide in 500 ml of dichloromethane. After stirring at room temperature for 24 hours the obtained N,N'-dicyclohexyl urea was separated by filtration and washed with dichloromethane. The filtrate and washing liquid were combined and subsequently extracted with a 2 N hydrochloric acid solution, a saturated sodium bicarbonate solution and twice with water. The dichloromethane was evaporated in vacuo and toluene was added to the residue. The obtained crystals were filtered off and washed with toluene. After crystallization from a mixture of dichloromethane and toluene, 19.6 g of t-butyl 7-benzamido-3-methyl-3-cephem-4-carboxylate 1-oxide were obtained.

PMR ($CDCl_3$): 1.56 (s, 9H); 2.14 (s, 3H); 3.11; 3.41; 3.52; 3.83 (ABq, 2H, J 18.5 Hz); 4.65 (br., 1H); 6.25 (dd, 1H, 4.5 and 9.5 Hz); 7.37—7.97 (m, 5H); 7.56 (d, 1H, J 9.5 Hz).

IR: 3400, 1770, 1715, 1685, 1520, 1030 $cm^{-1}$.

b. A solution of 40 g t-butyl 7-benzamido-3-methyl-3-cephem-4-carboxylate 1-oxide in 2 l of a 1:1 mixture of dichloromethane and acetic acid was cooled in an ice-bath. Under exposure to light of two tungsten lamps of 150 W, bromination was effected with a total amount of 28 g of N-bromosuccinimide, which was added portionwise over about two hours. The exposure was continued for 3 hours. The obtained reaction mixture was washed three times with water and then with a saturated solution of sodium

bicarbonate and again with water. The organic phase was treated with 5 g of activated charcoal and then dried over magnesium sulfate. After filtration the solvent was evaporated in vacuo and the residue mainly consisting of a mixture of the compounds mentioned herebelow, was separated into its components by means of preparative high pressure chromatography over silica gel, using as eluent a mixture of dichloromethane and acetone (12:1). The components obtained were: 10.4 g of t-butyl 7-benzamido-3-bromomethyl-3-cephem-4-carboxylate 1-oxide.

PMR ($CDCl_3$ + DMSO—$D_6$): 1.57 (s, 9H); 3.44; 3.74; 3.79; 4.09 (ABq, 2H, J 18 Hz); 4.26; 4.42; 4.51; 4.67 (ABq, 2H, J 10.5 Hz); 4.90 (d, 1H, J 4.8 Hz); 6.20 (dd, 1H, J 4.8 and 9.5 Hz); 7.35—7.96 (m, 5H); 7.85 (d, 1H, J 9.5 Hz);

IR: 3330, 1795, 1715, 1645, 1520, 1030 $cm^{-1}$;

and 8.7 g of t-butyl 7-benzamido-2-bromo-3-bromomethyl-3-cephem-4-carboxylate 1-oxide.

PMR ($CDCl_3$ + DMSO—$D_6$): 1.59 (s, 1H); 4.17; 4.34; 4.58; 4.75 (ABq, 2H, J 10.5 Hz); 5.34 (d, 1H, J 5 Hz); 5.73 (s, 2H); 6.35 (dd, 1H, J 5 and 9.5 Hz); about 7.34—7.98 (m, 5H); 7.71 (d, 1H, J 9.5 Hz).

IR: 3380, 1790, 1725, 1680, 1520, 1050 $cm^{-1}$.

c. 6.25 g of t-butyl 7-benzamido-3-bromomethyl-3-cephem-4-carboxylate 1-oxide were added to 25 ml of trifluoroacetic acid at room temperature. After stirring for 15 minutes at room temperature, the trifluoroacetic acid was evaporated in vacuo. After addition of 20 ml of dichloromethane evaporation to dryness was repeated. 25 ml of ether were added to the residue and the formed crystals were vacuum filtered, washed with ether and dried under vacuo.

There were obtained 5.77 g of 7-benzamido-3-bromomethyl-3-cephem-4-carboxylic acid 1-oxide as a slightly yellowish coloured solid.

PMR (DMSO—$D_6$): 3.95 (s, 2H); 4.49; 4.78 (ABq, 2H, J 10 Hz); 5.13 (d, 1H, J 4.5 Hz); 6.14 (dd, 1H, J 4.5 and 8 Hz); 7.4—8.1 (m, 5H); 8.56 (d, 1H, J 8 Hz).

IR: 3385, 3280, 2550, 1785, 1720, 1642, 1605, 1580, 1515, 998 $cm^{-1}$.

d. 12.5 g of t-butyl 7-benzamido-2-bromo-3-bromomethyl-3-cephem-4-carboxylate 1-oxide were stirred in 50 ml of trifluoroacetic acid at room temperature for 15 minutes. After evaporation to dryness in vacuo, 25 ml of dichloromethane were added and evaporation to dryness was repeated. A 1:1 mixture of ether and heptane was added to the residue. The formed crystals were vacuum filtered and washed with the same mixture. There were obtained 9.6 g of 7-benzamido-2-bromo-3-bromomethyl-3-cephem-4-carboxylic acid 1-oxide as an almost colourless solid.

PMR (DMSO—$D_6$): 4.55 (s, 2H); 5.52 (d, 1H, J 5.2 Hz); 6.18 (dd, 2H, J 5.2 and 7.5 Hz); 6.20 (s, 1H); about 7.4—8.1 (m, 5H); 8.90 (d, 1H, J 7.5 Hz).

IR: 3400, 3315, 2565, 1800, 1725, 1660, 1620, 1600, 1580, 1515, 1052, 997 $cm^{-1}$.

e. 258 mg of hexamethyldisilazane were added to a suspension of 0.97 g of 7-benzamido-3-bromomethyl-3-cephem-4-carboxylic acid 1-oxide in 30 ml of dry dichloromethane under a nitrogen atmosphere. The temperature of the mixture was kept at 20°C by a water-bath. After 2 hours of stirring, the slightly brown coloured solution was evaporated to dryness. The volatile components were removed at a pressure of 0.5 mm of mercury. There were obtained 1.05 g of trimethylsilyl 7-benzamido-3-bromomethyl-3-cephem-4-carboxylate 1-oxide.

PMR ($CDCl_3$): 0.39 (s, 9H); 3.47; 3.95 (ABq, 2H, J 18.7 Hz); 4.31; 4.93 (ABq, 2H, J 10.5 Hz); 4.71 (d, 1H, J 4.5 Hz); 6.37 (dd, 1H, J 4.5 and 9.5 Hz); 7.2—8.0 (m, 6H).

IR ($CHCl_3$): 3410, 1806, 1712, 1675, 1250, 1058, 1045, 843 $cm^{-}$1.

f. To a suspension of 0.29 g of 7-benzamido-2-bromo-3-bromomethyl-3-cephem-4-carboxylic acid 1-oxide in 10 ml of toluene kept under a pressure of 5 cm of mercury, 94 mg of hexamethyldisilazane in 6 ml of toluene were added dropwise over 15 minutes at room temperature. The mixture was then stirred for 1.5 hours under the same conditions, whereafter the toluene was evaporated at a pressure of 0.5 mm of mercury. There was obtained 0.31 g of trimethylsilyl 7-benzamido-2-bromo-3-bromomethyl-3-cephem-4-carboxylate 1-oxide as a yellow brown coloured solid.

PMR ($CDCl_3$): 0.39 (s, 9H); 4.26; 4.84 (ABq, 2H, J 10 Hz); 5.39 (d, 1H, J 4.5 Hz); 5.72 (s, 1H); 6.46 (dd, 1H, J 4.5 and 9.5 Hz); 7.3—8.0 (m, 6H).

IR ($CHCl_3$): 3420, 1810, 1712, 1678, 1253, 1053, 1010, 850 $cm^{-1}$.

## Example II

a. 27 g of N-bromosuccinimide were added portionwise over 3 hours to an ice-cold solution of 30 g of t-butyl 7-formamido-3-methyl-3-cephem-4-carboxylate 1-oxide in 1600 ml of a 1:1 mixture of acetic acid and dichloromethane under exposure to light of two tungsten lamps of 150 W. After a total exposure of 4 hours the reaction mixture was then washed 3 times with water, a saturated solution of sodium bicarbonate and again with water. The organic phase was treated with activated charcoal, dried over magnesium sulfate and evaporated to dryness after filtration. The residue was subjected to preparative high pressure chromatography using a 4:1 mixture of dichloromethane and acetone as eluent to obtain by evaporation to dryness of the suitable fractions: 10.7 g of t-butyl 3-bromomethyl-7-formamido-3-cephem-4-carboxylate 1-oxide.

PMR ($CDCl_3$ + a trace of DMSO—$D_6$): 1.54 (s, 9H); 3.78 (s, 2H); 4.38 and 4.65 (ABq, 2H, J 10 Hz); 4.90 (d, 1H, J 5 Hz); 5.99 (dd, 1H, J 5 and 9.5 Hz); 8.22 (d, 1H, J 9.5 Hz); 8.30 (s, 1H).

IR: 3310, 1785, 1720, 1690, 1520, 1025 $cm^{-1}$;

and 12.7 g of t-butyl 2-bromo-3-bromomethyl-7-formamido-3-cephem-4-carboxylate 1-oxide.

PMR ($CDCl_3$ + DMSO—$D_6$; 300 MHz): 1.57 (s, 9H); 4.35; 4.38; 4.61; 4.65 (ABq, 2H, J 10.5 Hz); 5.35 (d, 1H, J 5 Hz); 5.90 (s, 1H); 6.14 (dd, 1H, J 5 and 9 Hz); 8.28 (s, 1H); 8.46 (d, 1H, J 9 Hz).

IR: 3280, 1805, 1730, 1690, 1510, 1060 $cm^{-1}$.

b. 10 g of t-butyl 3-bromomethyl-7-formamido-3-cephem-4-carboxylate 1-oxide in 40 ml of trifluoroacetic acid were stirred for 15 minutes at room temperature. After evaporation to dryness under vacuo, 25 ml of dichloromethane were added and evaporation to dryness was repeated. Ether was added to the residue and the formed crystals were vacuum filtered and washed with ether. The almost colourless solid was dried in vacuo. There were obtained 8.54 g of 3-bromomethyl-7-formamido-3-cephem-4-carboxylic acid 1-oxide.

PMR (DMSO—$D_6$): 3.88 (s, 2H); 4.47; 4.77 (ABq, 2H, J 9.8 Hz); 5.00 (d, 1H, 4.5 Hz); 6.01 (dd, 1H, J 5.2 and 9.5 Hz); 8.25 (s, 1H); 8.48 (d, 1H, J 9.5 Hz); 10.60 (s, 1H).

IR: 3292, 2635, 2580, 1787, 1719, 1650, 1528, 1002, 993 $cm^{-1}$.

c. 10 g of t-butyl 2-bromo-3-bromomethyl-7-formamido-3-cephem-4-carboxylate 1-oxide in 40 ml of trifluoroacetic acid were stirred for 15 minutes at room temperature. The solvent was removed by evaporation in vacuo, 25 ml of dichloromethane were added to the residue and evaporation to dryness was repeated. A 1:1 mixture of heptane and ether was added to the residue whereafter the slightly yellow crystals were vacuum filtered and washed with the same solvent mixture. There were obtained 9.57 g of 2-bromo-3-bromomethyl-7-formamido-3-cephem-4-carboxylic acid 1-oxide.

PMR (DMSO-$D_6$): 4.55 (s, 2H); 5.45 (d, 1H, J 5.3 Hz); 6.13 (dd, 1H, J 5.3 and 9 Hz); 6.23 (s, 1H); 8.25 (s, 1H); 8.67 (d, 1H, J 9 Hz); 11.03 (s, 1H).

IR: 3350, 2920, 2550, 1800, 1725, 1685, 1510, 1059, 993 $cm^{-1}$.

d. According to the procedure of Example Ie, 0.60 g of 3-bromomethyl-7-formamido-3-cephem-4-carboxylic acid 1-oxide was converted by stirring for 2.5 hours at room temperature with 265 mg of hexamethyldisilazane to obtain 640 mg of trimethylsilyl 3-bromomethyl-7-formamido-3-cephem-4-carboxylate 1-oxide.

PMR ($CDCl_3$): 0.39 (s, 9H); 3.49; 3.96 (ABq, 2H, J 18 Hz); 4.34; 4.86 (ABq, 2H, J 10 Hz); 4.60 (d, 1H, J 4.5 Hz); 6.18 (dd, 1H, J 4.5 and 10.5 Hz); 7.25 (d, 1H, J 10.5 Hz); 8.42 (s, 1H).

IR ($CHCl_3$): 3498, 1802, 1700, 1252, 1055, 1045, 850 $cm^{-1}$.

e. To a suspension of 244 mg of 2-bromo-3-bromomethyl-7-formamido-3-cephem-4-carboxylic acid 1-oxide in 10 ml of dry toluene kept under a pressure of 5 cm of mercury by means of an oil-pump, a solution of 82 mg of hexamethyldisilazane in toluene was added dropwise over one hour. The reaction mixture was kept at 20°C with a water-bath. After the addition of the hexamethyldisilazane was complete, stirring was continued for one hour under the same conditions, whereafter the mixture was evaporated to dryness at a pressure of 0.5 mm of mercury. There were obtained 220 mg of trimethylsilyl 2-bromo-3-bromomethyl-7-formamido-3-cephem-4-carboxylate 1-oxide as a slightly yellow-brown coloured solid.

PMR ($CDCl_3$): 0.43 (s, 9H); 4.30; 4.90 (ABq, 2H, J 10.5 Hz); 5.33 (d, 1H, J 5 Hz); 5.73 (s, 1H); 6.32 (dd, 1H, J 5 and 9 Hz); 7.08 (d, 1H, J 9 Hz); 8.47 (s, 1H).

IR ($CHCl_3$): 3400, 1811, 1709, 1252, 1053, 999, 848 $cm^{-1}$.

## Example III

a. 3.05 g of N-bromosuccinimide were added portionwise over 1.75 hours to an ice-cold solution of 4.6 g of t-butyl 2-bromo-3-methyl-7-phenylacetamido-3-cephem-4-carboxylate 1-oxide in 350 ml of dichloromethane under the exposure to light of a tungsten lamp of 150 W. After the addition of N-bromosuccinimide was complete, the exposure was continued for one hour. The orange coloured solution was evaporated to dryness in vacuo at low temperature. The obtained residue was chromatographed over silica gel, using as initial eluent a 72:1 mixture of methylene chloride and acetone and then a 49:1 mixture of the same components. By evaporation to dryness of the suitable fractions there was obtained 1.4 g of t-butyl-2-bromo-3-bromomethyl-7-phenylacetamido-3-cephem-4-carboxylate 1-oxide.

PMR ($CDCl_3$): 1.55 (s, 9H); 3.58 (s, 2H); 4.16; 4.61 (ABq, 2H, J 11 Hz); 5.10 (d, 1H, J 5 Hz); 5.52 (s, 1H); 6.07 (dd, 1H, J 5 and 10 Hz); 6.76 (d, 1H, J 10 Hz); 7.26 (s, 5H).

IR: 3350, 3260, 1790, 1705, 1670, 1500, 1060 $cm^{-1}$.

b. A solution of 5.6 g of t-butyl 2-bromo-3-bromomethyl-7-phenylacetamido-3-cephem-4-carboxylate 1-oxide in 40 ml of trifluoroacetic acid was stirred for 15 minutes at room temperature. The solvent was removed in vacuo and ether was added to the residue, whereafter the evaporation to dryness was repeated. After addition of ether, the crystals were collected by filtration. There were obtained 4.72 g of 2-bromo-3-bromomethyl-7-phenylacetamido-3-cephem-4-carboxylic acid 1-oxide as a slightly yellow solid.

PMR (DMSO—$D_6$): 3.60 (s, 2H); 4.46 (s, 2H); 5.29 (d, 1H, 4.5 Hz); 5.87 (dd, 1H, J 4.5 and 7.5 Hz); 6.09 (s, 1H); 7.20 (s, 5H); 8.53 (d, 1H, J 7.5 Hz).

IR: 3340, 1790, 1730, 1705, 1620, 1525, 1040 $cm^{-1}$.

c. A solution of 37.3 g of t-butyl 3-bromomethyl-7-phenylacetamido-3-cephem-4-carboxylate 1-oxide in 130 ml of trifluoroacetic acid was stirred for 15 minutes at room temperature and then the solvent was removed by evaporation in vacuo. After addition of 100 ml of 1,2-dichloroethane, evaporation to dryness was repeated. Ether was added to the residue and the crystals were vacuum filtered, washed with ether and

dried in vacuo. There were obtained 33.7 g of 3-bromomethyl-7-phenylacetamido-3-cephem-4-carboxylic acid 1-oxide as an almost colourless solid.

PMR (DMSO-$D_6$): 3.52 and 3.80 (ABq, 2H, J 14.5 Hz); 3.85 (s, 2H); 4.46, 4.79 (ABq, 2H, J 10 Hz); 4.95 (d, 1H, J 4.5 Hz); 5.83 (dd, 1H, J 4.5 and 8 Hz); 7.33 (m, 5H); 8.42 (d, 1H, J 8 Hz).

IR: 3280, 3035, 1788, 1755, 1730, 1660, 1638, 1530, 1498, 1454, 1001, 709 $cm^{-1}$.

d. 258 mg of hexamethyldisilazane were added at room temperature to a suspension of 1.00 g of 3-bromomethyl-7-phenylacetamido-3-cephem-4-carboxylic acid 1-oxide in 30 ml of dry dichloromethane, substantially free of alcohol, under a nitrogen atmosphere. After 20 minutes, a clear, slighly brown coloured solution was obtained. The nitrogen atmosphere was sustained for 2 hours, whereafter the solvent and other volatile components were removed at a pressure of 0.5 mm of mercury. There was obtained 1.14 g of trimethylsilyl 3-bromomethyl-7-phenylacetamido-3-cephem-4-carboxylate 1-oxide as a yellow-brown powder.

PMR ($CDCl_3$): 0.30 (s, 9H); 3.31, 3.72 (ABq, 2H, J 18 Hz); 3.57 (s, 2H); 4.20, 4.75 (ABq, 2H, J 10.5 Hz); 4.43 (d, 1H, J 4.5 Hz); 6.00 (dd, 1H, J 4.5 and 9 Hz); 6.95 (d, 1H, J 9 Hz); 7.27 (s, 5H).

IR ($CHCl_3$): 3400, 3275, 1803, 1768, 1709, 1690, 1055, 1048, 848 $cm^{-1}$.

e. According to the procedure of Example IIe, 0.33 g of trimethylsilyl 2-bromo-3-bromomethyl-7-phenylacetamido-3-cephem-4-carboxylate 1-oxide was obtained as a slightly brown coloured solid from 0.30 g of 2-bromo-3-bromomethyl-7-phenylacetamido-3-cephem-4-carboxylic acid 1-oxide and 107 mg of hexamethyldisilazane.

PMR ($CDCl_3$) 0.36 (s, 9H); 3.64 (s, 2H); 4.20 and 4.78 (ABq, 2H, J 10.5 Hz); 5.17 (d, 1H, J 4.5 Hz); 5.58 (s, 1H); 6.17 (dd, 1H, J 4.5 and 9.5 Hz); 6.78 (d, 1H, J 9.5 Hz); 7.34 (s, 5H).

IR ($CHCl_3$): 3400, 1809, 1710, 1685, 1380, 1055, 998, 843 $cm^{-1}$.

## Example IV

a. A solution of 21.8 g of t-butyl 3-methyl-7-phenoxyacetamido-3-cephem-4-carboxylate 1-oxide in a mixture of 500 ml of dichloromethane and 500 ml of acetic acid under a nitrogen atmosphere was cooled in an ice-bath and exposed to the light of a tungsten lamp of 150 W. 9.7 g of N-bromosuccinimide were added and the mixture was exposed for 1.5 hours. Then the reaction mixture was subsequently washed twice with 1.5 l of water, 0.5 l of a saturated sodium bicarbonate solution and with 1 l of water. The organic phase was dried over magnesium sulfate and after filtration concentrated by evaporation to a volume of about 50 ml. There were obtained 3.2 g of t-butyl 2-bromo-3-bromomethyl-7-phenoxyacetamido-3-cephem-4-carboxylate 1-oxide by high pressure chromatography over silica gel and using a 12:1 mixture of dichloromethane and acetone, followed by evaporation to dryness of the suitable fractions.

PMR ($CDCl_3$): 1.58 (s, 9H); 4.23, 4.71 (ABq, 2H, J 10.5 Hz); 4.61 (s, 2H); 5.26 (d, 1H, J 5 Hz); 5.62 (s, 1H); 6.29 (dd, 1H, J 5 and 10.5 Hz); 6.8—7.6 (m, 5H); 7.90 (d, 1H, J 10.5 Hz).

IR ($CHCl_3$): 3385, 1810, 1727, 1703, 1505, 1490, 1380, 1370, 1150, 1060, 1000 $cm^{-1}$.

After evaporation of subsequent fractions, there were obtained 5.5 g of t-butyl 3-bromomethyl-7-phenoxyacetamido-3-cephem-4-carboxylate 1-oxide.

PMR ($CDCl_3$ + DMSO—$D_6$): 1.55 (s, 9H); 3.81 (s, 2H); 4.48 (s, 2H); 4.60 (s, 2H); 4.97 (d, 1H, J 4.5 Hz); 6.07 (dd, 1H, J 4.5 and 10 Hz); 6.8—7.5 (m, 5H); 8.08 (d, 1H, J 10 Hz).

IR: 3365, 1790, 1720, 1695, 1520, 1065 $cm^{-1}$.

b. 1.2 g of t-butyl 2-bromo-3-bromomethyl-7-phenoxyacetamido-3-cephem-4-carboxylate 1-oxide was stirred in 8 ml of trifluoroacetic acid for 15 minutes. After evaporation to dryness under vacuo, 20 ml of 1,2-dichloroethane were added to the residue and then the evaporation to dryness was repeated. 5 ml of ether and 50 ml of heptane were added to the residue and the obtained product was vacuum filtered and dried in vacuo. There was obtained 1.1 g of 2-bromo-3-bromomethyl-7-phenoxyacetamido-3-cephem-4-carboxylic acid 1-oxide as almost colourless crystals.

PMR (DMSO—$D_6$): 4.39 and 4.57 (ABq, 2H, J 10 Hz); 4.66 (s, 2H); 5.40 (d, 1H, J 4.8 Hz); 6.11 (dd, 1H, J 4.8 and 9 Hz); 6.18 (s, 1H); 6.7—7.5 (m, 5H); 8.24 (d, 1H, J 9 Hz).

IR: 3475, 1803, 1727, 1696, 1520, 1490, 1230, 1053, 996 $cm^{-1}$.

c. According to the procedure of Example IIIc, 2.0 g of 3-bromomethyl-7-phenoxyacetamido-3-cephem-4-carboxylic acid 1-oxide were obtained from 2.3 g of t-butyl 3-bromomethyl-7-phenoxyacetamido-3-cephem-4-carboxylate 1-oxide

PMR (DMSO—$D_6$): 3.87 (s, 2H); 4.44 and 4.72 (ABq, 2H, J 10 Hz); 4.66 (s, 2H); 5.01 (d, 1H, J 4.8 Hz); 6.03 (dd, 1H, J 4.8 and 9.5 Hz); 6.8—7.5 (m, 5H); 8.11 (d, 2H, J 9.5 Hz); about 10.5 (s, broad, 1H).

IR: 3275, 1780, 1770, 1722, 1675, 1530, 1233, 1218, 1175, 996 $cm^{-1}$.

d. According to the procedure of Example IIId, 600 mg of trimethylsilyl-3-bromomethyl-7-phenoxyacetamido-3-cephem-4-carboxylate 1-oxide were obtained from 525 mg of 3-bromomethyl-7-phenoxyacetamido-3-cephem-4-carboxylic acid 1-oxide and 138 mg of hexamethyldisilazane.

PMR ($CDCl_3$): 0.35 (s, 9H); 3.35 and 3.82 (ABq, 2H, J 18.5 Hz); 4.25 and 4.72 (ABq, 2H, J 10.5 Hz); 4.50 (s and d, 3H); 6.08 (dd, 1H, J 4.5 and 9.8 Hz); 6.7—7.5 (m, 5H); 7.82 (d, 1H, J 9.8 Hz).

IR ($CHCl_3$): 3380, 1807, 1702, 1388, 1372, 1255, 1062, 1050, 1003, 848 $cm^{-1}$.

e. According to the procedure of Example IIe, 300 mg of trimethylsilyl 2-bromo-3-bromomethyl-7-phenoxyacetamido-3-cephem-4-carboxylate 1-oxide were obtained as a slightly brown coloured solid from

330 mg of 2-bromo-3-bromomethyl-7-phenoxyacetamido-3-cephem-4-carboxylic acid 1-oxide and 101 mg of hexamethyldisilazane.

PMR ($CDCl_3$): 0.39 (s, 9H); 4.17 and 4.75 (ABq, 2H, J 10.5 Hz); 4.55 (s, 2H); 5.19 (d, 1H, J 5 Hz); 5.54 (s, 1H); 6.26 (dd, 1H, J 5 and 10 Hz); 6.7—7.5 (m, 5H); 7.77 (d, 1H, J 10 Hz).

IR ($CHCl_3$): 3390, 1810, 1710, 1700, 1380, 1253, 1060, 1000, 848 $cm^{-1}$.

## Example V

123 mg of tri-n-propylchlorosilane were added to a suspension of 312 mg of 7-benzamido-2-bromo-3-bromomethyl-3-cephem-4-carboxylic acid 1-oxide (prepared according to the procedure of Example Id) in 25 ml of 1,2-dichloroethane under a nitrogen atmosphere. Subsequently, a solution of 64 mg of triethylamine in 4 ml of 1,2-dichloroethane was added dropwise over 15 minutes under cooling in an ice-bath. The clear, slightly yellow coloured solution was stirred for two hours at room temperature. After removal of the solvent in vacuo, 7 ml of ether were added. The obtained suspension was centrifuged and the clear liquid layer was separated and evaporated to dryness in vacuo. The residue was kept at a pressure of 0.5 mm of mercury for half an hour. There were obtained 210 mg of tri-n-propylsilyl 7-benzamido-2-bromo-3-bromomethyl-3-cephem-4-carboxylate 1-oxide.

PMR ($CDCl_3$): 0.4—1.7 (m, 21H); 4.24 and 4.72 (ABq, 2H, J 10.5 Hz); 5.30 (d, 1H, J 4.8 Hz); 5.65 (s, 1H); 6.33 (dd, 1H, 4.8 and 9.5 Hz); 7.1—7.8 (m, 6H).

IR ($CHCl_3$): 3420, 1820, 1715, 1690, 1625, 1510, 1490, 1385, 1260, 1070, 1015, 840 $cm^{-1}$.

## Example VI

To a stirred suspension of 0.5 g of 7-benzamido-3-bromo-methyl-3-cephem-4-carboxylic acid 1-oxide (prepared according to the procedure of Example Ic) in 30 ml of a 1:1 mixture of toluene and 1,2-dichloroethane, a solution of 188 mg of t-butyldimethylchlorosilane in the same solvent mixture was added under a nitrogen atmosphere at −10°C. Subsequently, a solution of 109 mg of trimethylamine in 24 ml of the same solvent mixture was added dropwise over half an hour. Stirring was continued at −10°C for 15 minutes and at 20°C for 2 hours. After evaporation to dryness in vacuo, 10 ml of ether were added and the precipitate was separated by centrifugation. The obtained liquor was evaporated to dryness and after drying the residue under a pressure of 0.5 mm of mercury, there was obtained 0.1 g of t-butyldimethylsilyl 7-benzamido-3-bromomethyl-3-cephem-4-carboxylate 1-oxide.

PMR ($CDCl_3$): 0.38 (s, 6H); 0.97 (s, 9H); 3.45 and 3.92 (ABq, 2H, J 18 Hz); 4.24 and 4.86; 4.32 and 4.98 (2ABq, 2H, J resp. 10.5 and 12 Hz); 4.67 (d, 1H, J 4.5 Hz); 6.27 (dd, 1H, J 4.5 and 9 Hz); 7.1—7.9 (m, 6H).

IR ($CHCl_3$): 3410, 1805, 1710, 1675, 1630, 1505, 1485, 1385, 1260, 1050, 1005, 840 $cm^{-1}$.

## Example VII

According to the procedure of Example V, 0.26 g of trihexylsilyl 3-bromomethyl-7-formamido-3-cephem-4-carboxylate 1-oxide was obtained by reaction of 0.35 g of 3-bromomethyl-7-formamido-3-cephem-4-carboxylic acid 1-oxide (prepared according to the procedure of Example IIb) with 0.33 g of tri-hexylchlorosilane and 0.1 g of triethylamine in 1,2-dichloroethane.

PMR ($CDCl_3$): 0.3—1.8 (m, 39H), 3.38 and 3.85 (ABq, 2H, J 18 Hz); 4.15 and 4.84; 4.24 and 4.93 (2ABq, 2H, J resp. 10.5 and 12 Hz); 4.52 (d, 1H, J 4.5 Hz); 5.83 (dd, 1H, J 4.5 and 9.5 Hz); 7.18 (d, 1H, J 9.5 Hz); 8.20 (s, 1H).

IR($CHCl_3$): 3400, 1805, 1700, 1625, 1080, 1060, 1005 $cm^{-1}$.

## Example VIII

According to the procedure of Example V, 0.18 g of t-butyldimethylsilyl 3-bromomethyl-7-phenoxyacetamido-3-cephem-4-carboxylate 1-oxide was obtained starting from 0.31 g of 3-bromomethyl-7-phenoxyacetamido-3-cephem-4-carboxylic acid 1-oxide (prepared according to the procedure of Example IVc), 90 mg of t-butyldimethylchlorosilane and 59 mg of triethylamine in 1,2-dichloroethane.

PMR ($CDCl_3$): 0.36 (s, 6H); 0.95 (s, 9H); 3.35 and 3.82 (ABq, 2H, J 18 Hz); 4.25 and 4.63; 4.30 and 4.77 (2ABq, 2H, J resp. 10.5 and 12 Hz); 4.50 (s and d, 3H), 6.05 (dd, 1H, J 4.5 and 10 Hz); 6.7—7.4 (m, 5H); 7.85 (d, 1H, J 10 Hz).

IR ($CHCl_3$): 3395, 1810, 1710, 1700, 1260, 1075, 1060, 1015, 840 $cm^{-1}$.

## Example IX

According to the procedure of Example V, 0.12 g of chloromethyldimethylsilyl 3-bromomethyl-7-phenoxyacetamido-3-cephem-4-carboxylate 1-oxide was obtained starting from 0.40 g of 3-bromomethyl-7-phenoxyacetamido-3-cephem-4-carboxylic acid 1-oxide (prepared according to the procedure of Example IVc), 129 mg of chloromethyldimethyl-chlorosilane and 85 mg of triethylamine in 1,2-dichloroethane.

PMR ($CDCl_3$ + DMSO—$D_6$): 0.50 (s, 6H); 3.05 (s, 2H); 3.79 (s, broad, 2H); 4.42 and 4.73 (ABq, 2H, J 10.5 Hz); 4.56 (s, 2H); 4.90 (d, 1H, J 4.5 Hz); 6.06 (dd, 1H, J 4.5 and 10 Hz); 6.7—7.5 (m, 5H); 7.98 (d, 1H, J 10 Hz).

IR ($CHCl_3$): 3390, 1810, 1705, 1260, 1080, 1020, 855 $cm^{-1}$.

**Claims for the Contracting States: BE CH DE FR GB LU NL SE**

1. A bromo-substituted deacetoxycephalosporin derivative of the general formula:

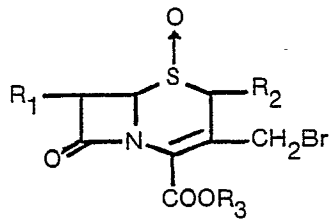

I

wherein $R_1$ represents a benzamido, formamido, phenylacetamido or phenoxyacetamido group, $R_2$ represents a hydrogen or bromine atom and $R_3$ represents a silyl group of the general formula:

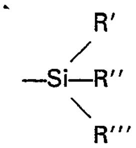

II

wherein R', R'' and R''' are the same or different and each represents an alkyl group (having at most 6 carbon atoms), a halogen substituted alkyl group (having at most 6 carbon atoms) or an aryl group, with the proviso that when $R_1$ represents a phenylacetamido group and $R_2$ represents a hydrogen or bromine atom, $R_3$ does not represent a trimethylsilyl group.

2. A compound according to claim 1 wherein $R_1$ represents a phenylacetamido group and $R_2$ represents a hydrogen or bromine atom, modified in that $R_3$ represents a trimethylsilyl group, in substantially pure form.

3. Trimethylsilyl 7-benzamido-3-bromomethyl-3-cephem-4-carboxylate 1-oxide.

4. Trimethylsilyl 7-benzamido-2-bromo-3-bromomethyl-3-cephem-4-carboxylate 1-oxide.

5. Trimethylsilyl 3-bromomethyl-7-formamido-3-cephem-4-carboxylate 1-oxide.

6. Trimethylsilyl 2-bromo-3-bromomethyl-7-formamido-3-cephem-4-carboxylate 1-oxide.

7. Trimethylsilyl 3-bromomethyl-7-phenoxyacetamido-3-cephem-4-carboxylate 1-oxide.

8. Trimethylsilyl 2-bromo-3-bromomethyl-7-phenoxyacetamido-3-cephem-4-carboxylate 1-oxide.

9. Tri-n-propylsilyl 7-benzamido-2-bromo-3-bromomethyl-3-cephem-4-carboxylate 1-oxide.

10. t-Butyldimethylsilyl 7-benzamido-3-bromomethyl-3-cephem-4-carboxylate 1-oxide.

11. Trihexylsilyl 3-bromomethyl-7-formamido-3-cephem-4-carboxylate 1-oxide.

12. t-Butyldimethylsilyl 3-bromomethyl-7-phenoxyacetamido-3-cephem-4-carboxylate 1-oxide.

13. Chloromethyldimethylsilyl 3-bromomethyl-7-phenoxyacetamido-3-cephem-4-carboxylate 1-oxide.

14. Process for the preparation of bromo-substituted deacetoxycephalosporins characterized by protecting the carboxyl group in cephalosporin sulphoxides of the general formula:

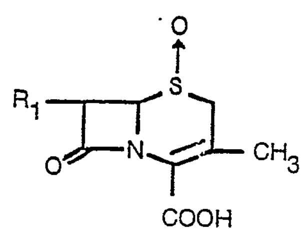

III

wherein $R_1$ is as defined in claim 1, with a group which can be split off easily, brominating the compounds thus protected to provide the corresponding 3'-bromo and 2,3'-dibromo-substituted compounds of the general formula:

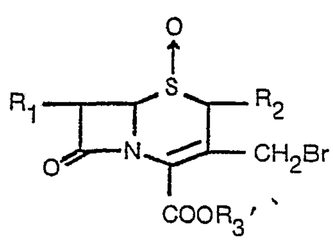

IV

wherein $R_1$ and $R_2$ are as defined in claim 1 and $R_3'$ represents a carboxyl-protecting group, subsequently hydrolyzing the deacetoxycephalosporins thus obtained to provide the corresponding compounds with a free carboxyl group of the general formula:

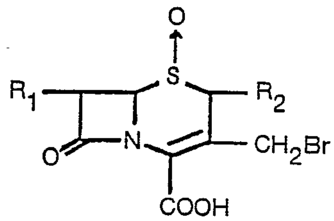

V

wherein $R_1$ and $R_2$ are as defined in claim 1, and silylating these compounds in known manner to provide the bromo-substituted deacetoxycephalosporins as defined in claim 1.

15. Use of the compound of claim 1 or claim 2 as intermediate in processes for the preparation of antibiotics of the class of cephalosporin derivatives.

**Claims for the Contracting State: AT**

1. Process for the preparation of a bromo-substituted deacetoxycephalosporin derivative of the general formula:

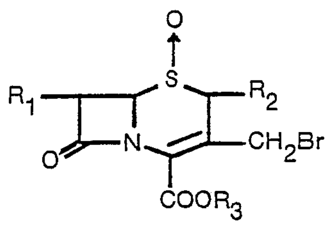

I

wherein $R_1$ represents a benzamido, formamido, phenylacetamido or phenoxyacetamido group, $R_2$ represents a hydrogen or bromine atom and $R_3$ represents silyl group of the general formula:

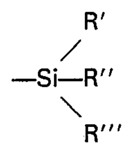

II

wherein R′, R″ and R‴ are the same or different and each represents an alkyl group (having at most 6 carbon atoms), a halogen substituted alkyl group (having at most 6 carbon atoms) or an aryl group, characterized by protecting the carboxyl group of a cephalosporin sulphoxide of the general formula:

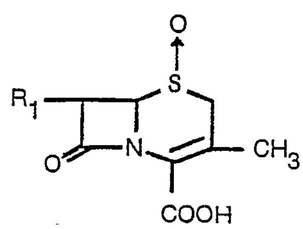

III

wherein $R_1$ is as hereinbefore defined, with a group which can be split off easily, brominating the compound thus protected to provide the corresponding 3′-bromo and 2,3′-dibromo-substituted compound of the general formula:

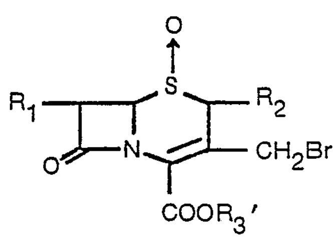

IV

wherein $R_1$ and $R_2$ are as hereinbefore defined and $R_3'$ represents a carboxyl-protecting group, subsequently hydrolyzing the deacetoxycephalosporin compound thus obtained to provide the corresponding compound with a free carboxyl group of the general formula:

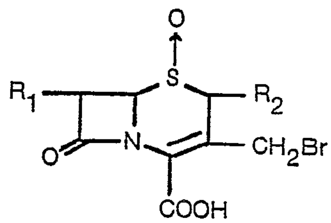

V

wherein $R_1$ and $R_2$are as hereinbefore defined, and silylating this compound in manner known per se to provide the bromo-substituted deacetoxycephalosporin derivative of formula I.

2. Use of the compound obtained from the process of claim 1 as intermediate in processes for the preparation of antibiotics of the class of cephalosporin derivatives.

**Claims for the Contracting State: IT**

1. A bromo-substituted deacetoxycephalosporin derivative of the general formula:

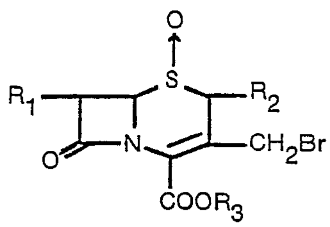

I

wherein $R_1$ represents a benzamido, formamido, phenylacetamido or phenoxyacetamido group, $R_2$ represents a hydrogen or bromine atom and $R_3$ represents a silyl group of the general formula:

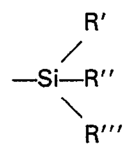

II

wherein R′, R″ and R‴ are the same or different and each represents an alkyl group (having at most 6 carbon atoms), a halogen substituted alkyl group (having at most 6 carbon atoms) or an aryl group.

2. Trimethylsilyl 7-benzamido-3-bromomethyl-3-cephem-4-carboxylate 1-oxide.
3. Trimethylsilyl 7-benzamido-2-bromo-3-bromomethyl-3-cephem-4-carboxylate 1-oxide.
4. Trimethylsilyl 3-bromomethyl-7-formamido-3-cephem-4-carboxylate 1-oxide.
5. Trimethylsilyl 2-bromo-3-bromomethyl-7-formamido-3-cephem-4-carboxylate 1-oxide.
6. Trimethylsilyl 3-bromomethyl-7-phenylacetamido-3-cephem-4-carboxylate 1-oxide.
7. Trimethylsilyl 2-bromo-3-bromomethyl-7-phenylacetamido-3-cephem-4-carboxylate 1-oxide.
8. Trimethylsilyl 3-bromomethyl-7-phenoxyacetamido-3-cephem-4-carboxylate 1-oxide.
9. Trimethylsilyl 2-bromo-3-bromomethyl-7-phenoxyacetamido-3-cephem-4-carboxylate 1-oxide.
10. Tri-n-propylsilyl 7-benzamido-2-bromo-3-bromomethyl-3-cephem-4-carboxylate 1-oxide.
11. t-Butyldimethylsilyl 7-benzamido-3-bromomethyl-3-cephem-4-carboxylate 1-oxide.
12. Trihexylsilyl 3-bromomethyl-7-formamido-3-cephem-4-carboxylate 1-oxide.
13. t-Butyldimethylsilyl 3-bromomethyl-7-phenoxyacetamido-3-cephem-4-carboxylate 1-oxide.
14. Chloromethyldimethylsilyl 3-bromomethyl-7-phenoxyacetamido-3-cephem-4-carboxylate 1-oxide.
15. Process for the preparation of bromo-substituted deacetoxycephalosporins characterized by protecting the carboxyl group in cephalosporin sulphoxides of the general formula:

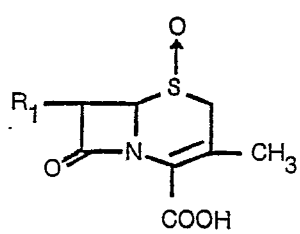

III

wherein $R_1$ is as defined in claim 1, with a group which can be split off easily, brominating the compounds

thus protected to provide the corresponding 3'-bromo and 2,3'-dibromo-substituted compounds of the general formula:

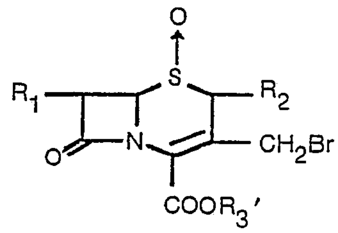

IV

wherein $R_1$ and $R_2$ are as defined in claim 1 and $R_3'$ represents a carboxy-protecting group, subsequently hydrolyzing the deacetoxycephalosporins thus obtained to provide the corresponding compounds with a free carboxyl group of the general formula:

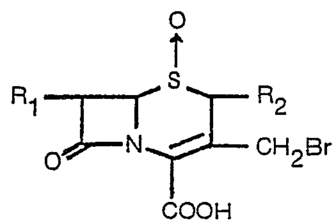

V

wherein $R_1$ and $R_2$ are as defined in claim 1, and silylating these compounds in known manner to provide the bromo-substituted deacetoxycephalosporins as defined in claim 1.

16. Use of the compound of claim 1 as intermediate in processes for the preparation of antibiotics of the class of cephalosporin derivatives.

**Patentansprüche für die Vertragsstaaten: BE CH DE FR GB LU NL SE**

1. Bromosubstituiertes Desacetoxycephalosporinderivat der allgemeinen Formel:

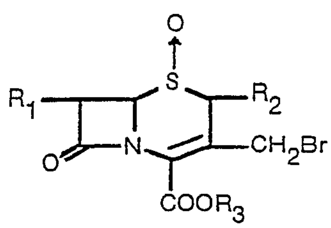

I

worin $R_1$ eine Benzamido-, Formamido-, Phenylacetamido- oder Phenoxyacetamidogruppe bedeutet, $R_2$ ein Wasserstoff- oder Bromatom bedeutet und $R_3$ eine Silylgruppe der allgemeinen Formel:

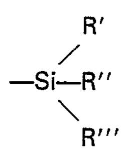

II

bedeutet, worin R', R'' und R''' gleich oder verschieden sind und jeweils eine Alkylgruppe (mit höchstens 6 Kohlenstoffatomen), eine halogensubstituierte Alkylgruppe (mit höchstens 6 Kohlenstoffatomen) oder eine Arylgruppe bedeuten, mit der Bedingung, dass $R_3$ keine Trimethylsilylgruppe darstellt, wenn $R_1$ eine Phenylacetamidogruppe bedeutet und $R_2$ ein Wasserstoff- oder Bromatom bedeutet.

2. Verbindung nach Anspruch 1, worin $R_1$ eine Phenylacetamidogruppe bedeutet und $R_2$ ein Wasserstoff- oder Bromatom bedeutet, dadurch modifiziert, dass $R_3$ eine Trimethylsilylgruppe bedeutet, in im wesentlichen reiner Form.

3. Trimethylsilyl-7-benzamido-3-bromomethyl-3-cephem-4-carboxylat-1-oxid.

4. Trimethylsilyl-7-benzamido-2-brom-3-brommethyl-3-cephem-4-carboxylat-1-oxid.

5. Trimethylsilyl-3-brommethyl-7-formamido-3-cephem-4-carboxylat-1-oxid.

6. Trimethylsilyl-2-brom-3-brommethyl-7-formamido-3-cephem-4-carboxylat-1-oxid.

7. Trimethylsilyl-3-brommethyl-7-phenoxyacetamido-3-cephem-4-carboxylat-1-oxid.

8. Trimethylsilyl-2-brom-3-brommethyl-7-phenoxyacetamido-3-cephem-4-carboxylat-1-oxid.

9. Tri-n-propylsilyl-7-benzamido-2-brom-3-brommethyl-3-cephem-4-carboxylat-1-oxid.

10. tert-Butyldimethylsilyl-7-benzamido-3-brommethyl-3-cephem-4-carboxylat-1-oxid.

11. Trihexylsilyl-3-brommethyl-7-formamido-3-cephem-4-carboxylat-1-oxid.

12. tert-Butyldimethylsilyl-3-brommethyl-7-phenoxyacetamido-3-cephem-4-carboxylat-1-oxid.

13. Chlormethyldimethylsilyl-3-brommethyl-7-phenoxyacetamido-3-cephem-4-carboxylat-1-oxid.

14. Verfahren zur Herstellung von bromsubstituierten Desacetoxycephalosporinen, dadurch gekennzeichnet, dass man die Carboxylgruppe in Cehalosporinsulfoxiden der allgemeinen Formel:

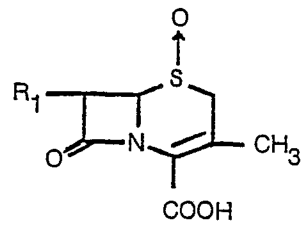

III

worin $R_1$ wie in Anspruch 1 definiert ist, mit einer Gruppe schützt, die leicht abgespalten werden kann, die so geschützten Verbindungen bromiert, um die entsprechenden 3'-brom- und 2,3'-dibrom-substituierten Verbindungen der allgemeinen Formel:

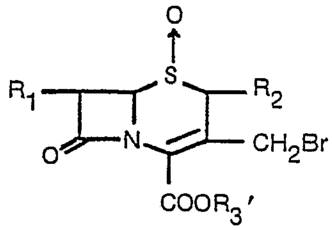

IV

worin $R_1$ und $R_2$ wie in Anspruch 1 definiert sind und $R_3'$ eine Carboxylschutzgruppe darstellt, herzustellen, die so erhaltenen Desacetoxycephalosporine anschliessend hydrolysiert, um die entsprechenden Verbindungen mit einer freien Carboxylgruppe der allgemeinen Formel:

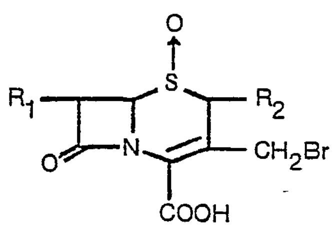

V

worin $R_1$ und $R_2$ wie in Anspruch 1 definiert sind, herzustellen, und diese Verbindungen in bekannter Weise silyliert, um die wie in Anspruch 1 definierten bromsubstituierten Desacetoxycephalosporine herzustellen.

15. Verwendung der Verbindung nach Anspruch 1 oder Anspruch 2 als Zwischenprodukt in Verfahren zur Herstellung von Antibiotica aus der Klasse der Cephalosporinderivate.

**Patentansprüche für den Vertragsstaat: AT**

1. Verfahren zur Herstellung eines bromsubstituierten Desacetoxycephalosporinderivatives der allgemeinen Formel:

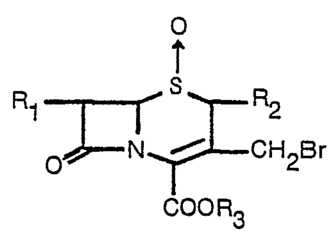

I

worin $R_1$ eine Banzamido-, Formamido-, Phenylacetamido- oder Phenoxyacetamidogruppe bedeutet, $R_2$ ein Wasserstoff- oder Bromatom bedeutet und $R_3$ eine Silylgruppe der allgemeinen Formel:

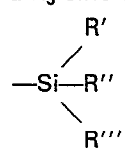

II

bedeutet, worin R′, R″ und R‴ gleich oder verschieden sind und jeweils eine Alkylgruppe (mit höchstens 6 Kohlenstoffatomen), eine halogensubstituierte Alkylgruppe (mit höchstens 6 Kohlenstoffatomen) oder eine Arylgruppe bedeuten, dadurch gekennzeichnet, dass man die Carboxylgruppe eines Cephalosporinsulfoxids der allgemeinen Formel:

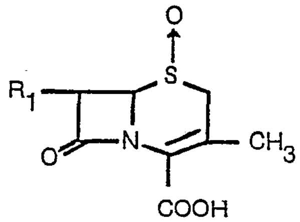

III

worin $R_1$ wie vorstehend definiert ist, mit einer Gruppe schützt, die leicht abgespalten werden kann, die so geschützte Verbindung bromiert, um die entsprechende 3′-brom- und 2,3′-dibrom-substituierte Verbindung der allgemeinen Formel:

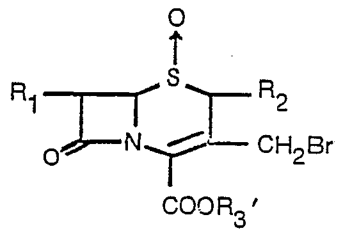

IV

worin $R_1$ und $R_2$ wie vorstehend definiert sind und $R_3'$ eine Carboxylschutzgruppe darstellt, zu erhalten, die so erhaltene Desacetoxycephalosporinverbindung anschliessend hydrolysiert, um die entsprechende Verbindung mit einer freien Carboxylgruppe der allgemeinen Formel:

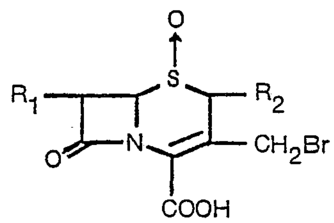

V

worin $R_1$ und $R_2$ wie vorstehend definiert sind, zu erhalten, und diese Verbindung in an sich bekannter Weise silyliert, um das bromsubstituierte Desacetoxycephalosporinderivat der Formel I zu erhalten.

2. Verwendung der aus dem Verfahren nach Anspruch 1 erhaltenen Verbindung als Zwischenprodukt in Verfahren zur Herstellung von Antibiotica aus der Klasse der Cehalosporinderivate.

**Patentansprüche für den Vertragsstaat: IT**

1. Bromsubstituiertes Desacetoxycephalosporinderivat der allgemeinen Formel:

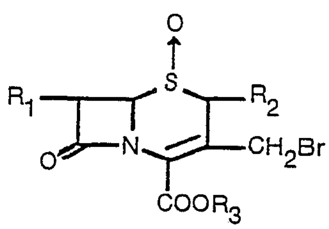

I

worin $R_1$ eine Benzamido-, Formamido-, Phenylacetamido- oder Phenoxyacetamidogruppe bedeutet, $R_2$ ein Wasserstoff- oder Bromatom bedeutet und $R_3$ eine Silylgruppe der allgemeinen Formel:

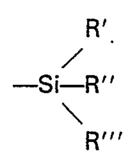

II

worin R′, R″ und R‴ gleich oder verschieden sind und jeweils eine Alkylgruppe (mit höchstens 6

Kohlenstoffatomen), eine halogensubstituierte Alkylgruppe (mit höchstens 6 Kohlenstoffatomen) oder eine Arylgruppe bedeuten.

2. Trimethylsilyl-7-benzamido-3-bromomethyl-3-cephem-4-carboxylat-1-oxid.
3. Trimethylsilyl-7-benzamido-2-brom-3-brommethyl-3-cephem-4-carboxylat-1-oxid.
4. Trimethylsilyl-3-brommethyl-7-formamido-3-cephem-4-carboxylat-1-oxid.
5. Trimethylsilyl-2-brom-3-brommethyl-7-formamido-3-cephem-4-carboxylat-1-oxid.
6. Trimethylsilyl-3-brommethyl-7-phenylacetamido-3-cephem-4-carboxylat-1-oxid.
7. Trimethylsilyl-2-brom-3-brommethyl-7-phenylacetamido-3-cephem-4-carboxylat-1-oxid.
8. Trimethylsilyl-3-brommethyl-7-phenoxyacetamido-3-cephem-4-carboxylat-1-oxid.
9. Trimethylsilyl-2-brom-3-brommethyl-7-phenoxyacetamido-3-cephem-4-carboxylat-1-oxid.
10. Tri-n-propylsilyl-7-benzamido-2-brom-3-brommethyl-3-cephem-4-carboxylat-1-oxid.
11. tert-Butyldimethylsilyl-7-benzamido-3-brommethyl-3-cephem-4-carboxylat-1-oxid.
12. Trihexylsilyl-3-brommethyl-7-formamido-3-cephem-4-carboxylat-1-oxid.
13. tert.-Butyldimethylsilyl-3-brommethyl-7-phenoxyacetamido-3-cephem-4-carboxylat-1-oxid.
14. Chlormethyldimethylsilyl-3-brommethyl-7-phenoxyacetamido-3-cephem-4-carboxylat-1-oxid.
15. Verfahren zur Herstellung von bromsubstituierten Desacetoxycephalosporinen, dadurch gekennzeichnet, dass man die Carboxylgruppe in Cehalosporinsulfoxiden der allgemeinen Formel:

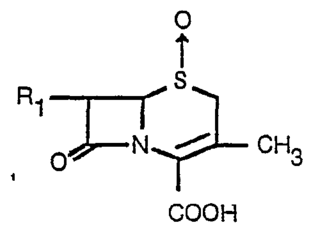

III

worin $R_1$ wie in Anspruch 1 definiert ist, mit einer Gruppe schützt, die leicht abgespalten werden kann, die so geschützten Verbindungen bromiert, um die entsprechenden 3′-brom- und 2,3′-dibrom-substituiert Verbindungen der allgemeinen Formel:

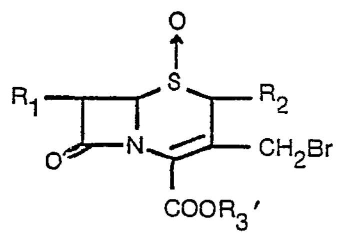

IV

worin $R_1$ und $R_2$ wie in Anspruch 1 definiert sind und $R_3'$ eine Carboxylschutzgruppe darstellt, zu erhalten, die so erhaltenen Desacetoxycephalosporine anschliessend hydrolysiert, um die entsprechenden Verbindungen mit einer freien Carboxylgruppe der allgemeinen Formel:

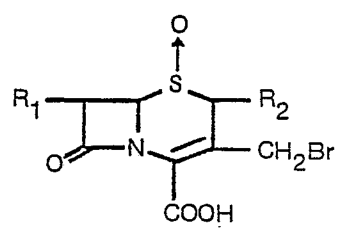

V

worin $R_1$ und $R_2$ wie in Anspruch 1 definiert sind, zu erhalten, und diese Verbindungen in bekannter Weise silyliert, um die wie in Anspruch 1 definierten bromsubstituierten Desacetoxycephalosporine zu erhalten.

16. Verwendung der Verbindung nach Anspruch 1 als Zwischenprodukt in Verfahren zur Herstellung von Antibiotika aus der Klasse der Cephalosporinderivate.

**Revendications pour les Etats contractants: BE CH DE FR GB LU NL SE**

1. Dérivé de désacetoxycéphalosporine bromo-substitué de la formule générale:

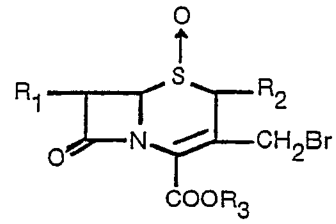

I

où $R_1$ représente un radical benzamido, formamido, phénylacétamido ou phénoxyacétamido, $R_2$ représente un atome d'hydrogène ou de brome et $R_3$ représente un radical silyle de la formule générale:

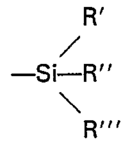

II

où R', R'' et R''' sont identiques ou différents et présentent chacun un radical alcoyle (comptant au maximum 6 atomes de carbone), un radical alcoyle halogéno substitué (comptant au maximum 6 atomes de carbone) ou un radical aryle, avec la restriction que lorsque $R_1$ représente un radical phénylacétamido et $R_2$ représente un atome d'hydrogène ou de brome, $R_3$ ne représente pas un radical triméthylsilyle.

2. Composé suivant la revendication 1, dans lequel $R_1$ représente un radical phénylacétamido et $R_2$ représente un atome d'hydrogène ou de brome, modifié en ce que $R_3$ représente un radical triméthylsilyle, sous forme sensiblement pure.

3. Le 1-oxyde de 7-benzamido-3-bromométhyl-3-céphème-4-carboxylate de triméthylsilyle.

4. Le 1-oxyde de 7-benzamido-2-bromo-3-bromométhyl-3-céphème-4-carboxylate de triméthylsilyle.

5. Le 1-oxyde de 3-bromométhyl-7-formamido-3-céphème-4-carboxylate de triméthylsilyle.

6. Le 1-oxyde de 2-bromo-3-bromométhyl-7-formamido-3-céphème-4-carboxylate de triméthylsilyle.

7. Le 1-oxyde de 3-bromométhyl-7-phénoxyacétamido-3-céphème-4-carboxylate de triméthylsilyle.

8. Le 1-oxyde de 2-bromo-3-bromométhyl-7-phénoxyacétamido-3-céphème-4-carboxylate de triméthylsilyle.

9. Le 1-oxyde de 7-benzamido-2-bromo-3-bromométhyl-3-céphème-4-carboxylate de tri-n-propylsilyle.

10. Le 1-oxyde de 7-benzamido-3-bromométhyl-3-céphème-4-carboxylate de t-butyldiméthylsilyle.

11. Le 1-oxyde de 3-bromométhyl-7-formamido-3-céphème-4-carboxylate de trihexylsilyle.

12. Le 1-oxyde de 3-bromométhyl-7-phénoxyacétamido-3-céphème-4-carboxylate de t-butyldiméthylsilyle.

13. Le 1-oxyde de 3-bromométhyl-7-phénoxyacétamido-3-céphème-4-carboxylate de chlorométhyldiméthylsilyle.

14. Procédé de préparation de désacétoxycéphalosporines bromo-substituées, caractérisé par la protection du radical carboxyle dans des sulfoxydes de céphalosporine de la formule générale:

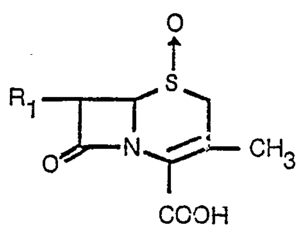

III

où $R_1$ est tel que défini dans la revendication 1, au moyen d'un radical qui peut être éliminé aisément, la bromation des composés ainsi protégés pour former les composés 3'-bromo-substitués et 2,3'-dibromo-substitués correspondants de la formule générale:

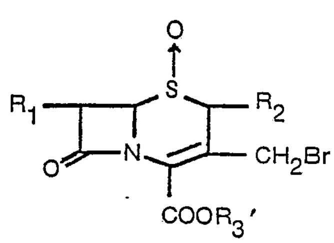

IV

où $R_1$ et $R_2$ sont tels que définis dans la revendication 1 et $R_3'$ représente un radical protecteur de la fonction carboxyle, ensuite l'hydrolyse des désacétoxycéphalosporines ainsi obtenues pour former les composés correspondants portant un radical carboxyle libre de la formule générale:

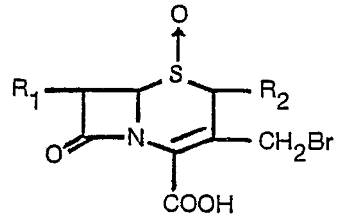

V

où $R_1$ et $R_2$ sont tels que définis dans la revendication 1, et la silylation de ces composés de manière connue pour former les désacétoxycéphalosporines bromo-substituées telles que définies dans la revendication 1.

15. Utilisation du composé suivant la revendication 1 ou 2 comme intermédiaire dans des procédés pour la préparation d'antiobiotiques de la classe des dérivés de la céphalosporine.

**Revendications pour l'Etat contractant: AT**

1. Procédé de préparation d'un dérivé de désacétoxycéphalosporine bromo-substitué de la formule générale:

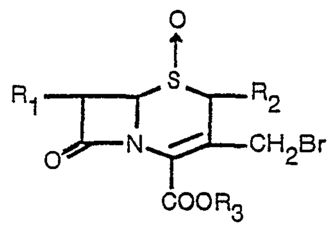

I

où $R_1$ représente un radical benzamido, formamido, phénylacétamido ou phénoxyacétamido, $R_2$ représente un atome d'hydrogène ou de brome et $R_3$ représente un radical silyle de la formule générale:

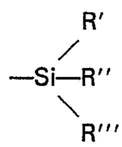

II

où R', R'' et R''' sont identiques ou différents et présentent chacun un radical alcoyle (comptant au maximum 6 atomes de carbone), un radical alcoyle halogéno substitué (comptant au maximum 6 atomes de carbone) ou un radical aryle, caractérise par la protection du radical carboxyle d'un sulfoxyde de céphalosporine de la formule générale:

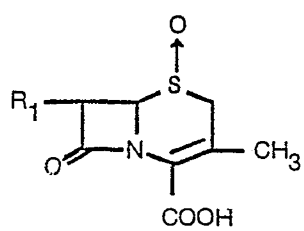

III

où $R_1$ est tel que défini ci-dessus, au moyen d'un radical qui peut être éliminé aisément, la bromation du composé ainsi protégé pour former les composés 3'-bromo-substitué et 2,3'-dibromo-substitué correspondants de la formule générale:

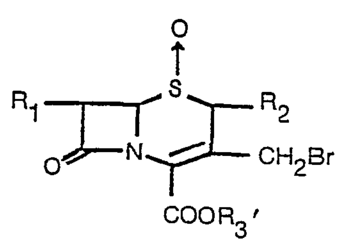

IV

où $R_1$ et $R_2$ sont tels que définis ci-dessus et $R_3'$ représente un radical protecteur de la fonction carboxyle, ensuite l'hydrolyse de la désacétoxycéphalosporine ainsi obtenue pour former le composé correspondant portant un radical carboxyle libre de la formule générale:

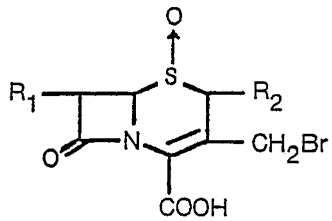

V

où $R_1$ et $R_2$ sont tels que définis ci-dessus et la silylation de ce composé de manière connue pour former le dérivé de désacétoxycéphalosporine bromo-substitué de formule I.

2. Utilisation du composé obtenue par le procédé suivant la revendication 1 comme intermédiaire dans des procédés pour la préparation d'antibiotiques de la classe des dérivés de la céphalosporine.

**Revendications pour l'Etat contractant: IT**

1. Dérivé de désacétoxycéphalosporine bromo-substitué de la formule générale:

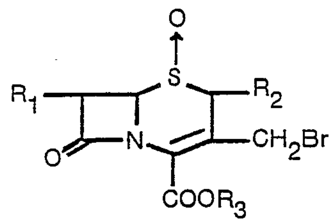

I

où $R_1$ représente un radical benzamido, formamido, phénylacétamido ou phénoxyacétamido, $R_2$ représente un atome d'hydrogène ou de brome et $R_3$ représente un radical silyle de la formule générale:

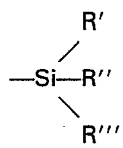

II

où R′, R″ et R‴ sont identiques ou différents et présentent chacun un radical alcoyle (comptant au maximum 6 atomes de carbone), un radical alcoyle halogéno substitué (comptant au maximum 6 atomes de carbone) ou un radical aryle.

2. Le 1-oxyde de 7-benzamido-3-bromométhyl-3-céphème-4-carboxylate de triméthylsilyle.

3. Le 1-oxyde de 7-benzamido-2-bromo-3-bromométhyl-3-céphème-4-carboxylate de triméthylsilyle.

4. Le 1-oxyde de 3-bromométhyl-7-formamido-3-céphème-4-carboxylate de triméthylsilyle.

5. Le 1-oxyde de 2-bromo-3-bromométhyl-7-formamido-3-céphème-4-carboxylate de triméthylsilyle.

6. Le 1-oxyde de 3-bromométhyl-7-phénylacétamido-3-céphème-4-carboxylate de triméthylsilyle.

7. Le 1-oxyde de 2-bromo-3-bromométhyl-7-phénylacétamido-3-céphème-4-carboxylate de triméthylsilyle.

8. Le 1-oxyde de 3-bromométhyl-7-phénoxyacétamido-3-céphème-4-carboxylate de triméthylsilyle.

9. Le 1-oxyde de 2-bromo-3-bromométhyl-7-phénoxyacétamido-3-céphème-4-carboxylate de triméthylsilyle.

10. Le 1-oxyde de 7-benzamido-2-bromo-3-bromométhyl-3-céphème-4-carboxylate de tri-n-propylsilyle.

11. Le 1-oxyde de 7-benzamido-3-bromométhyl-3-céphème-4-carboxylate de t-butyldiméthylsilyle.

12. Le 1-oxyde de 3-bromométhyl-7-formamido-3-céphème-4-carboxylate de trihexylsilyle.

13. Le 1-oxyde de 3-bromométhyl-7-phénoxyacétamido-3-céphème-4-carboxylate de t-butyldiméthylsilyle.

14. Le 1-oxyde de 3-bromométhyl-7-phénoxyacétamido-3-céphème-4-carboxylate de chlorométhyldiméthylsilyle.

15. Procédé de préparation de désacétoxycéphalosporines bromo-substituées, caractérisé par la protection du radical carboxyle dans des sulfoxydes de céphalosporine de la formule générale:

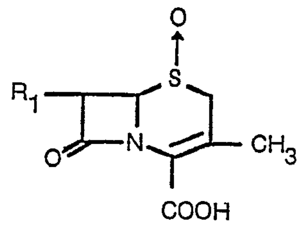

III

où $R_1$ est tel que défini dans la revendication 1, au moyen d'un radical qui peut être éliminé aisément, la bromation des composés ainsi protégés pour former les composés 3'-bromo-substitués et 2,3'-dibromo-substitués correspondants de la formule générale:

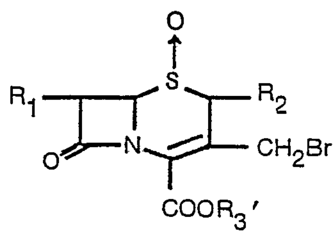

IV

où $R_1$ et $R_2$ sont tels que définis dans la revendication 1 et $R_3'$ représente un radical protecteur de la fonction carboxyle, ensuite l'hydrolyse des désacétoxycéphalosporines ainsi obtenues pour former les composés correspondants portant un radical carboxyle libre de la formule générale:

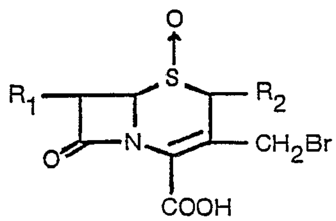

V

où $R_1$ et $R_2$ sont tels que définis dans la revendication 1, et la silylation de ces composés de manière connue pour former les désacétoxycéphalosporines bromo-substituées telles que définies dans la revendication 1.

16. Utilisation du composé suivant la revendication 1 comme intermédiaire dans des procédés pour la préparation d'antibiotiques de la classe des dérivés de la céphalosporine.